# EUROPÄISCHE PATENTSCHRIFT

(11) **EP 0 657 170 B1**
(45) Veröffentlichungstag und Bekanntmachung des Hinweises auf die Patenterteilung: **15.03.2000**
(21) Anmeldenummer: 94118466.5
(22) Anmeldetag: 24.11.1994
(51) Int. Cl.: A61K 38/04, A61K 38/09, A61K 9/00, A61K 9/22, A61K 9/52, A61K 9/19, A61J 1/00

(54) **Erzeugnisse zur Anwendung von initial hohen Dosen von Cetrorelix und Herstellung einer Kombinationspackung zur Verwendung bei Therapie von Krankheiten**
Products for the application of high initial doses of cetrorelix and preparation of a combined package for use in treating diseases
Produits pour l'application de doses initiales élevées de cétrorelix et préparation d'un emballage combiné pour l'utilisation dans la thérapie de maladies

(30) Priorität: 09.12.1993 DE 4342091
(43) Veröffentlichungstag der Anmeldung: 14.06.1995
(73) Patentinhaber: ASTA Medica Aktiengesellschaft, 01277 Dresden (DE)
(72) Erfinder: Engel, Jürgen, Prof., D-63755 Alzenau (DE); Hilgard, Peter, Dr., D-60325 Frankfurt (DE); Reissmann, Thomas, Dr., D-60437 Frankfurt (DE)
(74) Vertreter: Decker, Karl-Ludwig

(56) Entgegenhaltungen:
- EP-A- 0 089 548
- EP-A- 0 132 102
- EP-A- 0 171 550
- EP-A- 0 237 159
- EP-A- 0 268 066
- EP-A- 0 294 493
- EP-A- 0 299 402
- EP-A- 0 315 414
- EP-A- 0 442 671
- WO-A-90/03182
- WO-A-90/11070
- AT-T- 81 285
- DE-A- 2 335 265
- DE-A- 2 729 068
- DE-A- 2 834 226
- DE-A- 2 840 461
- GB-A- 1 204 580
- US-A- 4 305 502
- E. KORKUT ET AL.: "Inhibition of growth of experimental prostate cancer with sustained delivery systems (microcapsules and microgranules) of the luteinizing hormone-releasing hormone antagonist SB-75" PROCEEDINGS OF THE NATIONAL ACADEMY OF SCIENCES OF THE UNITED STATES OF AMERICA, Bd. 88, Nr. 1, 1. Januar 1991, Seiten 844-848, XP002081673
- AUSTRIA CODEX 1993/1994 FACHINFORMATION, Bd. 1, 1993, Seite 808 XP002081674 Österreichische Apotheker Verlagsgesellschaft MBH, WIEN;
- AUSTRIA CODEX 1993/1994 FACHINFORMATION, Bd. 2, 1993, Seiten 2144-2147, XP002081675 Österreichische Apotheker Verlagsgesellschaft MBH, WIEN
- AUSTRIA CODEX 1993/1994 FACHINFORMATION, Bd. 3, 1993, XP002081676 Österreichische Apotheker Verlagsgesellschaft MBH, WIEN

## Beschreibung

Zur Therapie hormonabhängiger maligner Erkrankungen stehen zur Zeit die Substanzen Zoladex® (INN: Goserelin) und Decapeptyl® (INN: Triptorelin) zur Verfügung.
Zoladex® wird in Form eines länglichen Zylinders von 1 cm Länge und 1 mm Durchmesser mit einem speziellen Applicator unter die Haut gespritzt.
Decapeptyl® liegt in Form einer Microkapsel-Emulsion vor, die ebenfalls subcutan gegeben wird. Beide Formen stellen eine kontinuierliche Wirkstoffabgabe an das umgebende Gewebe sicher. Der Wirkmechanismus beider Substanzen ist der eines Superagonisten.
Die Substanz Cetrorelix (INN) ist ein Antagonist von LHRH. Der Wirkmechanismus unterscheidet sich vollkommen von dem der bekannten Superagonisten. Synthese und einige wichtige pharmakologische Wirkungen sind in EP 299 402 beschrieben.
Daher benötigt man zur Therapie mit Cetrorelix andere Dosen.
Zur vollkommenen Unterdrückung der Hormonkonzentration auf Kastrationsniveau ist eine Dosis von 10 mg täglich im Probandenversuch notwendig. Diese hohe Tagesdosis läßt sich nicht in Retard-Formen, die über einen längeren Zeitraum, beispielsweise mehrere Monate, wirken sollen, unterbringen.
Das unter die Haut zu spritzende Depot wurde zu voluminös und wäre nicht mehr verträglich. Es besteht also die Aufgabe ein Erzeugnis zur sicheren und verträglichen Applikation zur Verfügung zu stellen.
Beim DMBA-induzierten Mamma-Carcinom an der Ratte wurde nun gefunden, daß bei einer initial hohen Dosis und einer weiteren Dosis, die für sich allein gegeben nicht aktiv ist, ein Behandlungserfolg zu erzielen war.

Der überraschende Tierbefund konnte auch am Menschen bestätigt werden.
Im Probandenversuch wurde gefunden, daß nach der Injektion einer Anfangsdosis von 10 mg, die zu einer Suppression von LH, FSH und Testosteron führte, bei Erhaltungsdosen von 1 mg alle 12 Stunden, 2 mg alle 24 Stunden und 1 mg alle 24 Stunden ebenfalls eine Suppression von LH, FSH und Testosteron zu beobachten war. Der Versuch wurde über drei Wochen durchgeführt.
Die Erhaltungsdosen, die oben gegeben wurden, sind alleine gegeben nicht geeignet, die Suppression von LH, FSH und Testosteron zu erreichen.

Damit eröffnet sich eine einfache und sichere Lösung der Aufgabe, in dem man Arzneimittelpackungen, die die aktive Substanz
- in der Initialdosis mit der Menge von 1 - 60 mg in einer Lyophilisat-Ampulle enthalten und die Erhaltungsdosis entweder in einer oder mehreren
   - Lyophilisat-Ampullen mit einer Retard-Form mit einer Abgaberate von 0,1 - 10 mg/Tag für die gesamte Therapiedauer enthalten oder
   - Lyophilisat-Ampullen mit der Menge an aktiver Substanz, die nicht in retardierter Form vorliegt, in der Menge von 0,1 - 10 mg enthalten,
zur Verfügung stellt.

Ähnliche Erzeugnisse zur Anwendung in der Therapie von Tumorerkrankungen mit Alkylantien vom Oxazaphosphorin-Typ, die das detoxifizierende Agens Mesna in einer Kombinationspackung mit dem Cytostatikum enthalten, sind aus der EP-A-000 2495 bekannt.
Dort kommt es darauf an, dem Arzt ein Erzeugnis zur sicheren Vermeidung der urotoxischen Nebenwirkungen der Oxazaphosphorin-Cytostatika zur Verfügung zu stellen.

Hier stellt sich eine andere Aufgabe. Es wird nicht ein Arzneimittelerzeugnis zur Verfügung gestellt, welches die Nebenwirkungen einer cytostatischen Therapie zu unterdrücken in der Lage ist, sondern es wird ein Arzneimittelerzeugnis zur Verfügung gestellt, welches die Initialdosis und die erforderliche Erhaltungsdosis dem Arzt in einer Packung zur Verfügung stellt.
Dadurch wird erreicht, daß
- die Therapiesicherheit steigt,
- Verwechslungen im Klinikalltag vermieden werden und
- die Compliance erhöht wird.

Darüberhinaus besteht die Möglichkeit, durch den geringeren Einsatz an biologisch aktiver Substanz die Therapiekosten zu senken und die Nebenwirkungen zu reduzieren.

Die Initialdosis liegt im Erzeugnis als leichtlösliches Salz, beispielsweise als Acetat, vor.
Das Acetat wird beispielsweise folgendermaßen hergestellt:

In ein geeignetes Glasgefäß werden ca. 1,5 Liter Wasser für Injektionszwecke vorgelegt. In ein weiteres Glasgefäß wurden 210 g Wasser für Injektionsszwecke vorgelegt und 91,17 g Essigsäure zugefügt. Die berechnete Menge Cetrorelixacetat (1,62 - 1,695 g, je nach Gehalt der eingesetzten Charge) wird in der hergestellten 30 %igen Essigsäure unter Rühren gelöst. Diese Lösung wird in das Glasgefäß mit 1,5 Liter Wasser für Injektionszwecke überführt, 82,2 g Mannitol zugegeben, gelöst und mit Wasser für Injektionszwecke auf 3039 g aufgefüllt.

### Inprozeßkontrollen:

- pH-Wert:: 2,5 - 3,0
- Dichte:: 1,009 - 1,017 g/cm³ bei 20°C
- Berechnungsindex:: 1,227 - 1,340 bei 440 nm und 20°C

Die Sterilisationen der Lösung erfolgt durch Filtration über ein geeignetes Membranfilter (Porenweite 0,2 µm, unter aseptischen Bedingungen). 100 ml Vorlauf sind zu verwerfen.
Die Filter sind mit gespanntem Wasserdampf zu sterilisieren. Cetrorelix Gefriertrocknungslösung wird vor Rekontamination geschützt aufbewahrt.
Die Lösung wird unverzüglich in Injektionsflaschen DIN 2R farblos, hydrolytische Klasse I unter aseptischen Bedingungen dosiert und mit sterilen Gefriertrocknungsstopfen versehen. Die Sollfüllmenge beträgt 2,0 ml = 2,026 g

Die 2 ml Injektionsflaschen wurden vorher auf einer Injektionsflaschenwaschmaschine gespült und mit Heißluft getrocknet und sterilisiert. Die gereinigten Gefriertrocknungsstopfen wurden autoklaviert. Die vorverschlossenen Injektionsflaschen wurden in eine Gefriertrocknungsanlage überführt und bei einer Plattentemperatur von -40°C auf +20°C steigend lyophilisiert. Anschließend wird die Anlage mit sterilem Stickstoff geflutet, die Flaschen in der Anlage verschlossen und die Stopfen mit Bördelklappen gesichert.

Die Injektionsflaschen werden visuell auf Verschlußfehler und äußere Fehler kontrolliert. Fehlerhafte Injektionsflaschen werden aussortiert und vernichtet.

Cetrorelix Lyophilisat 1 mg ist ein weißer, fester Gefriertrocknungskuchen in einer farblosen 2 ml Injektionsflasche, die mit grauen Gefriertrocknungsstopfen und gelber Flipp-off Bördelkappe verschlossen ist.

Die Erhaltungsdosis kann im Erzeugnis ebenfalls als Acetat, als Embonat oder als Einbettung in Micropartikel gemäß DE-OS 42 23 282.1 oder E. Korkut, L. Bokser, A.M. Comaru-Schally, K. Groot and A.V. Schally:Inhibition of growth of experimental prostate cancer with sustained delivery systems (microcapsules and microgranules) of the luteinizing hormone-releasing hormone antagonist SB-75, Proc. Natl. Accad. Sci. USA, Vol. 88, pp. 844-848 (1991) formuliert werden.
Das Embonat wird beispielsweise folgendermaßen hergestellt:

Im äquimolaren Verhältnis von Peptid (berechnet als freie Base) zu Embonsäure werden eine wäßrige, Alkali im Überschuß enthaltende Lösung von Embonsäure mit der essigsauren Cetrorelixacetatlösung vereinigt, wobei die Embonsäure als gelbe Kristalle präzipiert. Bei Zugabe von verdünnter Natronlauge bis pH 7 - 7,5 löst sich die Embonsäure und fällt mit dem Dekapeptid als weißes Cetrorelixembonatsalz der molaren Zusammensetzung Peptid: Embonsäure 2:1 (Mol/Mol) aus. Der Niederschlag wird abfiltriert, mit H₂O gewaschen und getrocknet.

Initialdosis und die Erhaltungsdosis werden in einer Umverpackung so zusammengepackt, daß eine ausreichende Substanzmenge für beispielsweise eine einwöchige oder eine einmonatliche Therapie bereitgestellt wird.
Eine Packung für die Monatstherapie beispielsweise ist folgendermaßen dimensioniert:
- Ein Behälter ist mit der Initialdosis an Cetrorelix-Acetat-Lyophilisat gefüllt. Die Füllmenge beträgt zwischen 1 mg und 60 mg Lyophilisat
- Bis zu 30 weitere Behälter sind mit der Erhaltungsdosis an Cetrorelix-Acetat-Lyophilisat gefüllt.
   Die Füllmenge liegt zwischen 0,1 mg und 10 mg.
   Die Erhaltungsdosis kann auch als Retardformulierung vorliegen.

Die erfindungsgemäße Arzneimittelpackung kann Anwendung finden bei der Therapie von Prostatakrebs, Endometriose, benigner Prostatahypertrophie, Mamma-Carcinom, Ovarial-Carcinom, Uterus-Fibrom, Pubertas praecox. Die erfindungsgemäße Packung kann Anwendung finden bei männlicher Verhütung.

## Patentansprüche

1. Arzneimittelpackung, dadurch gekennzeichnet, daß sie in dem einen Behältnis die Initialdosis und in einem oder mehreren anderen Behältnissen die Erhaltungsdosis eines LH-RH Antagonisten enthält, wobei die Initialdosis zwischen 1 und 60 mg pro Tag liegt und höher ist als die Erhaltungsdosis, die zwischen 0,1 und 10 mg pro Tag liegt und für sich allein nicht ausreichend aktiv ist.

2. Arzneimittelpackung gemäß Anspruch 1, dadurch gekennzeichnet, daß der LH-RH Antagonist ein Lyophilisat des Cetrorelix ist.

3. Arzneimittelpackung gemäß Anspruch 2, dadurch gekennzeichnet, daß der LH-RH Antagonist, welcher als Erhaltungsdosis verwendet wird, eine Retardform von Cetrorelix ist.

4. Arzneimittelpackung gemäß Anspruch 3, dadurch gekennzeichnet, daß der LH-RH Antagonist, welcher als Erhaltungsdosis verwendet wird, als Cetrorelix-Embonat vorliegt.

5. Verfahren zur Herstellung einer Arzneimittelpackung gemäß Anspruch 2, dadurch gekennzeichnet, daß man eine Lösung von Cetrorelix Lyophilisat in die Behältnisse abfüllt, anschließend lyophilisiert und die Behältnisse verschließt.

6. Arzneimittelpackung gemäß Anspruch 1 zur Verwendung in der Therapie von Prostata-Krebs.

7. Arzneimittelpackung gemäß Anspruch 1 zur Verwendung in der Therapie von Endometriose.

8. Arzneimittelpackung gemäß Anspruch 1 zur Verwendung in der Therapie von benigner Prostatahypertrophie.

9. Arzneimittelpackung gemäß Anspruch 1 zur Verwendung in der Therapie von Mamma-Carcinom.

10. Arzneimittelpackung gemäß Anspruch 1 zur Verwendung in der Therapie von Ovarial-Carcinom.

11. Arzneimittelpackung gemäß Anspruch 1 zur Verwendung in der Therapie von Uterus-Fibrom.

12. Arzneimittelpackung gemäß Anspruch 1 zur Verwendung in der Therapie von Pubertas praecox.

13. Packung zur Verwendung bei männlicher Verhütung, dadurch gekennzeichnet, daß sie in dem einen Behältnis die Initialdosis und in einem oder mehreren anderen Behältnissen die Erhaltungsdosis eines LH-RH Antagonisten enthält, wobei die Initialdosis zwischen 1 und 60 mg pro Tag liegt und höher ist als die Erhaltungsdosis die zwischen 0,1 und 10 mg pro Tag liegt und für sich allein nicht ausreichend aktiv ist.

## Claims

1. Pharmaceutical pack, characterized in that it contains, in one container, the initial dose and, in one or more other containers, the maintenance dose of an LH-RH antagonist, where the initial dose is between 1 and 60 mg per day and is higher than the maintenance dose, which is between 0.1 and 10 mg per day and is not sufficiently active by itself.

2. Pharmaceutical pack according to Claim 1, characterized in that the LH-RH antagonist is a lyophilizate of cetrorelix.

3. Pharmaceutical pack according to Claim 2, characterized in that the LH-RH antagonist, which is used as a maintenance dose, is a delayed-release form of cetrorelix.

4. Pharmaceutical pack according to Claim 3, characterized in that the LH-RH antagonist, which is used as maintenance dose, is present as cetrorelix embonate.

5. Process for the production of a pharmaceutical pack according to Claim 2, characterized in that the solution of cetrorelix lyophilizate is dispensed into the containers and then lyophilized, and the containers are sealed.

6. Pharmaceutical pack according to Claim 1 for use in the therapy of prostate cancer.

7. Pharmaceutical pack according to Claim 1 for use in the therapy of endometriosis.

8. Pharmaceutical pack according to Claim 1 for use in the therapy of benign prostrate hypertrophy.

9. Pharmaceutical pack according to Claim 1 for use in the therapy of mastocarcinoma.

10. Pharmaceutical pack according to Claim 1 for use in the therapy of ovarian carcinoma.

11. Pharmaceutical pack according to Claim 1 for use in the therapy of uterine fibroma.

12. Pharmaceutical pack according to Claim 1 for use in the therapy of pubertas praecox.

13. Pack for use in male prophylaxis, characterized in that it contains, in one container, the initial dose, and in one or more other containers, the maintenance dose of an LH-RH antagonist, where the initial dose is between 1 and 60 mg per day and is higher than the maintenance dose, which is between 0.1 and 10 mg per day and is not sufficiently active by itself.

## Revendications

1. Conditionnement de médicament,
caractérisé en ce qu'
il renferme dans l'un des récipients, la dose initiale et dans un ou plusieurs autres récipients la dose d'entretien d'un antagoniste de LH-RH, dans lequel la dose initiale se situe entre 1 et 60 mg par jour et est plus élevée que la dose d'entretien qui se situe entre 0,1 et 10 mg par jour et qui n'est pas suffisamment active pour elle-même toute seule.

2. Conditionnement de médicament selon la revendication 1,
caractérisé en ce que
l'antagoniste de LH-RH est un lyophilisat de Cetrorelix.

3. Conditionnement de médicament selon la revendication 2,
caractérisé en ce que
l'antagoniste de LH-RH qui est utilisé en tant que dose d'entretien est une forme retard de Cetrorelix.

4. Conditionnement de médicament selon la revendication 3,
caractérisé en ce que
l'antagoniste de LH-RH, qui est utilisé en tant que dose d'entretien, se présente sous forme d'embonate de Cetrorelix.

5. Procédé de préparation d'un conditionnement de médicament selon la revendication 2,
caractérisé en ce qu'
on remplit une solution de lyophilisat de Cetrorelix dans les récipients.

6. Conditionnement de médicament selon la revendication 1, en vue de l'utilisation dans la thérapeutique du cancer de la prostate.

7. Conditionnement de médicament selon la revendication 1, en vue de l'utilisation dans la thérapeutique de l'endométriose.

8. Conditionnement de médicament selon la revendication 1, en vue de l'utilisation dans la thérapeutique de l'hypertrophie bénigne de la prostate.

9. Conditionnement de médicament selon la revendication 1, en vue de l'utilisation dans la thérapeutique du carcinome mammaire.

10. Conditionnement de médicament selon la revendication 1, en vue de l'utilisation dans la thérapeutique du carcinome des ovaires.

11. Conditionnement de médicament selon la revendication 1, en vue de l'utilisation dans la thérapeutique du fibrome de l'utérus.

12. Conditionnement de médicament selon la revendication 1, en vue de l'utilisation dans la thérapeutique de la puberté précoce.

13. Conditionnement en vue de l'utilisation dans la prévention masculine,
caractérisé en ce qu'
il renferme dans l'un des récipients la dose initiale et dans un ou plusieurs autres récipients la dose d'entretien d'un antagoniste de LH-RH, pour lequel la dose initiale se situe entre 1 et 60 mg par jour et est plus élevée que la dose d'entretien qui se situe entre 0,1 et 10 mg par jour et qui n'est pas suffisamment active pour elle toute seule.
